# EUROPEAN PATENT APPLICATION

(11) **EP 4 834 955 A1**
(43) Date of publication of application: **04.11.2026**
(21) Application number: 24909830.2
(22) Date of filing: 14.08.2024
(51) Int. Cl.: C08F 10/00, C08F 110/06, C08F 4/649

(54) **INTERNAL ELECTRON DONOR, SOLID CATALYST COMPONENT, POLYMERIZATION CATALYST, AND USE THEREOF**

(30) Priority: 27.12.2023 CN 202311822654
(71) Applicant: China Energy Investment Corporation Limited, Beijing 100011 (CN); National Institute of Clean-and-Low-Carbon Energy, Beijing 102211 (CN)
(72) Inventor: HUANG, Jinhua, Beijing 102211 (CN); YU, Peiqian, Beijing 102211 (CN); YU, Yue, Beijing 102211 (CN); FENG, Liuhai, Beijing 102211 (CN); SHI, Mengxue, Beijing 102211 (CN); LI, Yulian, Beijing 102211 (CN); BIAN, Yufei, Beijing 102211 (CN); ZHENG, Zhigang, Beijing 102211 (CN); CHEN, Linfeng, Beijing 102211 (CN)
(74) Representative: Bergenstråhle & Partners AB
(86) International application number: PCT/CN2024/111969
(87) International publication number: WO 2025/138879

(57) **Abstract**

The present disclosure relates to the technical field of olefin polymerization catalysts, and particularly to an internal electron donor, a solid catalyst component, a polymerization catalyst and use thereof in olefin polymerization. The internal electron donor of the olefin polymerization catalyst is at least one of compounds represented by Formula (I), wherein R₁ to R₁₁ are the same or different, and each is independently selected from H, halogen, C₁-C₁₀ alkyl, cycloalkyl, alkenyl and phenyl; halogenated or heteroatom substituted alkyl, cycloalkyl, phenyl, alkylphenyl, phenylalkyl, indenyl and benzyl, wherein the heteroatom is selected from N, O, S, P and Si; two or more of the R₁ and R₉ may be linked together to form cyclic ring(s) or unsaturated bond(s). A Ziegler-Natta polymerization catalyst prepared by using the compound provided by the present disclosure as an internal electron donor achieves the advantages of high catalytic activity and good hydrogen response, and the obtained polymer achieves a high isotactic index, a relatively low xylene-soluble fraction, and broad molecular weight distribution.

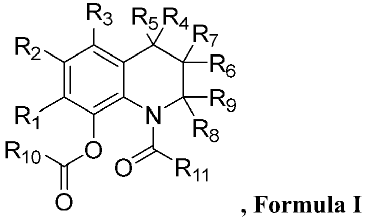

## Description

### TECHNICAL FIELD

The present disclosure relates to the technical field of olefin polymerization catalysts, and particularly to an internal electron donor, a solid catalyst component, a polymerization catalyst and use thereof in olefin polymerization.

### BACKGROUND

It is well known that the Ziegler-Natta catalysts used for olefin polymerization consist of three components: a magnesium chloride support, an internal electron donor compound, and a titanium compound. The internal electron donor compound can not only enhance the catalytic activity of the olefin polymerization catalyst, but also improve the stereoselectivity of the catalyst. Catalyst without internal electron donors exhibits significantly lower catalytic activity, and the resulting polymers fails to meet the requirements of practical application due to low isotactic index.

Up to the present, a variety of compounds have widely been used as internal electron donors for the preparation of the Ziegler-Natta catalysts. For example, aromatic monoesters or diesters such as diisobutyl phthalate or ethyl benzoate, etc., disclosed in US4,784,983 A; diol esters disclosed in CN 1453298 A; succinate esters disclosed in CN1313869A; diethers disclosed in EP 361,494 A; aromatic 1,2-phenylene diesters disclosed in US 61,141,902 A; and substituted amidobenzoates disclosed in EP 15,186,252 A/CN 108570120 A, etc., are all used as internal electron donors.

In industrial production, these internal electron donor compounds exhibit certain deficiencies in practical applications. For example, phthalate compounds, as plasticizer substances, have aroused growing concerns due to their potential adverse impacts on human health, which accordingly restricts their use in Ziegler-Natta catalysts. Catalysts using aromatic diester-based internal electron donors exhibit relatively low catalytic activity. Diether-based internal electron donors can endow catalyst with high catalytic activity and good hydrogen response, whereas the resultant polymers exhibit a rather narrow molecular weight distribution. Catalysts using aromatic 1,2-phenylene diester-based internal electron donors exhibit both good catalytic activity and hydrogen response, but yield polymers with a high xylene-soluble fraction. Catalysts employing substituted amidobenzoate-based internal electron donors exhibit low catalytic activity and a high xylene-soluble fraction.

Owing to the vital role of internal electron donors in catalysts and inherent deficiencies of existing internal electron donors in practical application, the improvement for internal electron donors remains a key research focus in this field.

Therefore, it is of great significance to develop a new olefin polymerization catalyst that can overcome the above-mentioned shortcomings in the prior art.

### SUMMARY OF THE INVENTION

Targeting at the above-mentioned problems in prior art, the object of the present disclosure is to provide an internal electron donor for an olefin polymerization catalyst, a solid catalyst component, an olefin polymerization catalyst, and use thereof. A polymerization catalyst prepared using the novel compound as an internal electron donor for the Ziegler-Natta catalyst exhibits the advantages of high catalytic activity and good hydrogen response, and polymer obtained by using the polymerization catalyst in olefin polymerization has the characteristics of high isotactic indexes, relatively low xylene-soluble fraction, and broad molecular weight distribution.

In order to achieve the above object, the present disclosure provides the following technical solutions.

According to a first aspect, the present disclosure provides an internal electron donor for an olefin polymerization catalyst, wherein the internal electron donor is at least one selected from the compounds represented by Formula I below: 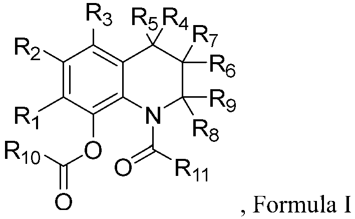 in Formula 1:
R₁ to R₉ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl; two or more of R₁ to R₉ may be linked together to form cyclic structure(s) or unsaturated bond(s);
R₁₀ and R₁₁ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl;
any hydrogen atoms on the benzene ring of the above-mentioned aryl, arylalkyl, or alkylaryl groups may each be optionally substituted with a halogen atom.

In some embodiments, in Formula I, each of R₁₀ and R₁₁ is independently selected from phenyl, tert-butyl, and ethoxy.

In some embodiments, in Formula I, preferably each of R₄, R₆, and R₈ is hydrogen with respect to the substituent pairs R₄ and R₅, R₆ and R₇, and R₈ and R₉.

According to the internal electron donor provided by the present disclosure, in some embodiments, the internal electron donor is at least one selected from the compounds represented by Formula (II) below: 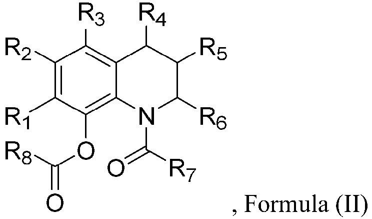 in Formula (II):
R₁ to R₆ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl; any two or more of R₁ to R₆ may be linked together to form cyclic ring(s) or unsaturated bond(s);
R₇ and R₈ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl.

According to the internal electron donor provided by the present disclosure, in some embodiments, in Formula (II), R₇ and R₈ are each independently selected from phenyl, tert-butyl and ethoxy.

According to a second aspect, the present disclosure provides a solid catalyst component for olefin polymerization, comprising: a titanium compound, a magnesium compound, and at least one of the above-mentioned internal electron donors.

In some embodiments, a method for preparing the solid catalyst component comprises: bring a magnesium compound precursor, a titanium compound and at least one of the internal electron donors into contact and reaction to obtain the solid catalyst component; wherein,
the magnesium compound precursor is at least one selected from compounds represented by Formula XₙMg(OR)₂₋ₙ, compounds represented by Formula MgCl₂·mROH, MgCl₂/SiO₂ mixtures, MgCl₂/Al₂O₃ mixtures, and mixtures of magnesium halides and titanium alkoxides, wherein m ranges from 0.1 to 6 (e.g., 0.2, 0.5, 0.8, 1, 2, 3, 4, or 5), 0≦n≦2 (e.g., n is 0, 1, or 2), X is selected from halogen (e.g., fluorine, chlorine, or bromine), R is selected from hydrogen and C₁-C₈ hydrocarbyl group (e.g., methyl, ethyl, n-propyl, n-butyl, isobutyl, 1-pentyl, or 1-hexyl);
the titanium compound is represented by Formula TiXₙ(OR)₄₋ₙ, wherein R is selected from C₁-C₂₀ hydrocarbyl, X is selected from halogen, and n ranges from 1 to 4 (e.g., 1, 2, 3, or 4).

In some embodiments, non-limiting examples of the titanium compound include titanium tetrachloride, titanium tetrabromide, titanium tetraiodide, or alkyl titanium halide. For example, alkyl titanium halide may be selected from methoxy titanium trichloride, ethoxy titanium trichloride, propoxy titanium trichloride, n-butoxy titanium trichloride, dimethoxy titanium dichloride, diethoxy titanium dichloride, dipropoxy titanium dichloride, di-n-butoxy titanium dichloride, trimethoxy titanium chloride, triethoxy titanium chloride, tripropoxy titanium chloride, or tri-n-butoxy titanium chloride. These alkyl titanium halides may be used alone or in combination.

In the present disclosure, the magnesium compounds are well-known in the art, and will not be elaborated in detail. For example, the magnesium compound may be derived from the magnesium compound precursor.

In the present disclosure, the preparation method and specific process for the solid catalyst component are conventional in the art, and will not be described in detail.

The content or amount of the internal electron donor in the solid catalyst component may be selected within conventional ranges known in the art.

According to a third aspect, the present disclosure provides an olefin polymerization catalyst, wherein the olefin is CH₂=CHR, wherein R is selected from hydrogen and C₁-C₁₂ hydrocarbyl group, the catalyst comprises a product obtained by reacting the following starting materials:
(a) at least one of the above-mentioned solid catalyst components;
(b) at least one of organo-aluminum compounds represented by Formula AlRₙX₍₃₋ₙ₎, wherein R is selected from hydrogen and C₁-C₂₀ hydrocarbyl group; X is selected from halogen, and n is an integer satisfying 0≦n≦3;
(c) at least one of alkoxysilane compounds represented by Formula RₙSi(OR¹)₄₋ₙ (as the external electron donor compound), wherein R and R¹ are the same or different, and each is independently selected from C₁-C₁₈ hydrocarbyl group and halogenated hydrocarbyl group, and C₁-C₁₀ substituent optionally containing heteroatom(s) (such as N, O, S, or P); and n is an integer satisfying 0≦n≦3.

In some embodiments, for the organo-aluminum compounds represented by Formula AlRₙX₍₃₋ₙ₎, each R is hydrogen or a C₁-C₁₂ hydrocarbyl group.

In some embodiments, the organo-aluminum compound may be an alkyl aluminum compound, such as a trialkylaluminum compound, and preferably one or more selected from trimethylaluminum, triethylaluminum, triisobutylaluminum, tri-n-butylaluminum, tri-n-hexylaluminum, and trioctylaluminum.

The amount of the organo-aluminum compound as a co-catalyst employed in the olefin polymerization catalysts is within conventional ranges well known in the art. For example, the molar ratio of the solid catalyst component to the organo-aluminum compound, Ti/Al, is 1:5 to 1:400.

In some embodiments, for the alkoxysilane compound represented by Formula RₙSi(OR¹)₄₋ₙ, each of R and R¹ is independently selected from C₁-C₁₈ alkyl, C₃-C₁₈ cycloalkyl, C₆-C₁₈ aryl, and C₁-C₁₈ haloalkyl.

The alkoxysilane compound may specifically include but is not limited to tetramethoxysilane, tetraethoxysilane (TEOS), dimethyldimethoxysilane, dimethyldiethoxysilane, methyl tert-butyldimethoxysilane, methyl isopropyldimethoxysilane, diphenyloxydimethoxysilane, vinyltrimethoxysilane, methylcyclohexyldimethoxysilane, ethylcyclohexyldimethoxysilane, di-n-propyldimethoxysilane, diisopropyldimethoxysilane, di-n-butyldimethoxysilane, diisobutyldimethoxysilane, di-tert-butyldimethoxysilane, dicyclopentyldimethoxysilane (D donor), cyclopentyltrimethoxysilane, isopropyltrimethoxysilane, n-propyltrimethoxysilane, n-propyltriethoxysilane, ethyltriethoxysilane, diethylaminotriethoxysilane, cyclohexylpyrrolidinyl dimethoxysilane, bis(pyrrolidinyl)dimethoxysilane, bis(perhydroisoquinolinyl)dimethoxysilane, 2-ethylpiperidinyl 2-tert-butyldimethoxysilane, (1,1,1-trifluoro-2-propyl)(2-ethylpiperidinyl) dimethoxysilane, and (1,1,1-trifluoro-2-propyl)(methyl)dimethoxysilane, etc. The alkoxysilane compound is preferably selected from dicyclopentyldimethoxysilane (D donor), methylcyclohexyldimethoxysilane, diisopropyldimethoxysilane, diisobutyldimethoxysilane, n-propyltrimethoxysilane, n-propyltriethoxysilane, tetraethoxysilane (TEOS), and diethylaminotriethoxysilane.

The amount of the alkoxysilane compound as an external electron donor component employed in the olefin polymerization catalysts is within conventional ranges well known in the art, and thus detailed descriptions are omitted herein.

According to a fourth aspect, the present disclosure provides use of the above-mentioned polymerization catalyst in olefin polymerization.

In the present disclosure, the olefin (which is CH₂=CHR, wherein R is hydrogen or a C₁-C₁₂ alkyl) may undergo either homopolymerization or copolymerization of ethylene and an alpha-olefin monomer. The polymerization process may adopt conventional procedures known in the art, and thus detailed descriptions are omitted herein.

Compared with the prior art, the technical solution of the present disclosure achieves at least as following beneficial effects: the present catalyst adopts the hydrogenated 8-hydroxyquinoline derivative as internal electron donor, and a Ziegler-Natta polymerization catalyst prepared using the novel compound as internal electron donor exhibits high catalytic activity and good hydrogen response; and the resultant polymer using the catalyst has the characteristics of high isotactic indexes, relatively low xylene-soluble fraction, and broad molecular weight distribution.

### EXAMPLES

For a better understanding of the technical features and merits of the present disclosure, preferred embodiments thereof are described in detail below. Although the preferred embodiments of the present disclosure have been described in the examples, it should be understood that the present disclosure can be implemented in various forms and should not be limited by the embodiments set forth herein.

Unless otherwise specified, all experimental procedures described in the Examples are performed in accordance with conventional procedures well known in the art.

The following starting materials and reagents used therein are all commercially available.

Details for part of the starting materials are listed below:
Internal electron donor C1, DNBP (di-n-butyl phthalate), commercially available;
Internal electron donor C2 (systematic name: 3-methyl-5-tert-butyl-1,2-di(benzoyl)benzenediol ester with the structure as shown in Table 1 or 2), commercially available from Beijing InnoChem Science & Technology Co., Ltd.;
Internal electron donor C3 (systematic name: phenyl 2-(N-methylbenzamido)benzoate with the structure as shown in Table 1 or 2), commercially available from Beijing InnoChem Science & Technology Co., Ltd.;
Internal electron donors ID1 to ID6 were all laboratory-synthesized, with their respective structures shown in Table 1 or 2.
Substituted or unsubstituted 8-hydroxy-1,2,3,4-tetrahydroquinoline, benzoyl chloride, o-methylbenzoyl chloride, ethyl chloroformate, etc., all purchased from Beijing InnoChem Science & Technology Co., Ltd.

Based on the structures shown in Table 1 or 2, the internal electron donors used in the Examples have the systematic names listed below:
internal electron donor ID1: N-benzoyl-8-benzoylhydroxy-1,2,3,4-tetrahydroquinoline;
internal electron donor ID2: N-benzoyl-7-methyl-8-benzoylhydroxy-1,2,3,4-tetrahydroquinoline;
internal electron donor ID3: N-benzoyl-5-tert-butyl-7-methyl-8-benzoylhydroxy-1,2,3,4-tetrahydroquinoline;
internal electron donor ID4: N-benzoyl-5-adamantyl-7-methyl-8-benzoylhydroxy-1,2,3,4-tetrahydroquinoline;
internal electron donor ID5: N-o-methylbenzoyl-8-o-methylbenzoylhydroxy-1,2,3,4-tetrahydroquinoline;
internal electron donor ID6: N-ethoxycarbonyl-8-ethoxyacyloxy-1,2,3,4-tetrahydroquinoline.

### Preparation of Internal Electron Donors

### Preparation Example 1

The synthesis procedure for internal electron donor ID1: 14.9 g (100 mmol) of 8-hydroxy-1,2,3,4-tetrahydroquinoline was charged into a 250 mL single-necked flask, followed by addition of 100 mL of tetrahydrofuran and 40 mL (300 mmol) of triethylamine. Under ice-water bath cooling, 23 mL (200 mmol) of benzoyl chloride was slowly added dropwise into the reaction mixture. After the completion of the dropwise addition, the mixture was allowed to react at room temperature for 3 hours, then the reactants were filtered, and the resulting mother liquor was concentrated, the resultant solid was washed three times with 50 mL of methanol, then recrystallized from ethyl acetate to give 31.3 g of the product ID1 with a yield of 87%.

### Preparation Example 2

The synthesis procedure for internal electron donor ID2 was identical to that described in Preparation Example 1, except that 8-hydroxy-1,2,3,4-tetrahydroquinoline was replaced with 7-methyl-8-hydroxy-1,2,3,4-tetrahydroquinoline.

### Preparation Example 3

The synthesis procedure for internal electron donor ID3 was identical to that described in Preparation Example 1, except that 8-hydroxy-1,2,3,4-tetrahydroquinoline was replaced with 5-tert-butyl-7-methyl-8-hydroxy-1,2,3,4-tetrahydroquinoline.

### Preparation Example 4

The synthesis procedure for internal electron donor ID4 was identical to that described in Preparation Example 1, except that 8-hydroxy-1,2,3,4-tetrahydroquinoline was replaced with 5-adamantyl-7-methyl-8-hydroxy-1,2,3,4-tetrahydroquinoline.

### Preparation Example 5

The synthesis procedure for internal electron donor ID5 was identical to that described in Preparation Example 1, except that the benzoyl chloride was replaced with o-methylbenzoyl chloride.

### Preparation Example 6

The synthesis procedure for internal electron donor ID6 was identical to that described in Preparation Example 1, except that the benzoyl chloride was replaced with ethyl chloroformate.

### Preparation of Catalysts and Polymers

### Catalyst Preparation Example 1

A custom-made 250 ml 6-necked reactor equipped with a stirrer and a filter unit was thoroughly purged with nitrogen gas, followed by charging 2.2 g of diethoxymagnesium and 50 mL of chlorobenzene. The system was maintained at -10°C and 100 mL of titanium tetrachloride/chlorobenzene solution (with a volume ratio of 1:1) was added dropwise. After completion of the addition, the system was slowly heated to 90°C, then a chlorobenzene solution of 1.8 g of internal electron donor ID1 was injected into the reactor with a syringe. The mixture was further heated to 110°C and held for 1 hour. Thereafter the mother liquor was filtered thoroughly with pressure. Another 100 mL of titanium tetrachloride/chlorobenzene solution (with a volume ratio of 1:1) was added dropwise, then the solution was allowed to react at a constant temperature of 110°C for 1 hour before pressure filtration again. Finally 100 mL of titanium tetrachloride/chlorobenzene solution (with a volume ratio of 1:1) was added dropwise and the mixture was allowed to react at a constant temperature of 110°C for another 0.5 hours, followed by complete removal of mother liquor by pressure filtration. After the collected solid was treated three times with washing using 100 mL of n-heptane and filtration, the product was dried to afford the solid catalyst component 1 as a solid powder.

The contents of titanium and internal electron donor in the solid catalyst component 1, and the structure of the internal electron donor are shown in Tables 1 and 2 below.

Olefin Polymerization Example a1:
Propylene polymerization was carried out in a 2L stainless steel autoclave in laboratory:
Step 1, the autoclave was purged with high-purity nitrogen (H₂O <1 ppm and O₂ <1 ppm); then, 0.55 L of liquid propylene and 0.2 g of hydrogen were charged sequentially at room temperature, and the system was subsequently heated to 70°C;
Step 2: prior to the autoclave temperature reaching 70°C, 0.75 ml of 1M triethylaluminum in heptane solution, 1.1 ml of 0.05M D-donor in hexane solution, and 6 mg of the solid catalyst component 1 (i.e., Z-N catalyst containing the internal electron donor ID1) prepared above were pre-complexed for 5 minutes. Once the temperature of the autoclave reached 70°C, the mixture obtained from the pre-complexation was charged into the autoclave and polymerization timing was initiated;
Step 3, after 60 minutes of reaction, the autoclave was vented and cooled down, and the reaction was quenched; the resulting polypropylene resin was discharged from the autoclave, then vacuum dried at 30°C for 2 hours to afford the polypropylene product.

The resultant polypropylene product was characterized to determine its polymer properties, and the test results are shown in Table 2.

### Olefin Polymerization Example b1

Propylene polymerization was carried out in a 2L stainless steel autoclave in laboratory:
Step 1, the autoclave was purged with high-purity nitrogen (H₂O <1 ppm, O₂ <1 ppm); then, 0.55 L of liquid propylene and 1.2 g of hydrogen were charged sequentially at room temperature, and the system was subsequently heated to 70°C;
Step 2: prior to the autoclave temperature reaching 70°C, 0.75 ml of 1M triethylaluminum in heptane solution, 1.1 ml of 0.05M D-donor in hexane solution, and 6 mg of the solid catalyst component 1 (i.e., Z-N catalyst containing the internal electron donor ID1) prepared above were pre-complexed for 5 minutes. Once the temperature of the autoclave reached 70°C, the mixture obtained from the pre-complexation was charged into the autoclave and polymerization timing was initiated;
Step 3, after 60 minutes of reaction, the autoclave was vented and cooled, and the reaction was quenched; the polypropylene resin was discharged from the autoclave and vacuum dried at 30°C for 2 hours to afford the polypropylene product.

The obtained polypropylene product was characterized to determine its polymer properties, and the test results are shown in Table 2.

### Catalyst Preparation Example 2

The synthetic procedure for the solid catalyst component was identical to that described in Catalyst Preparation Example 1, except that the internal electron donor ID1 was replaced with ID2. All remaining operating steps were kept consistent with Catalyst Preparation Example 1 to afford solid catalyst component 2 (i.e., Z-N catalyst containing internal electron donor ID2).

The contents of titanium and internal electron donor in the solid catalyst component 2, and the structure of the internal electron donor are shown in Tables 1 and 2 below.

### Olefin Polymerization Example a2

Propylene polymerization was carried out in accordance with Polymerization Example a1, except that the solid catalyst component 1 (i.e., Z-N catalyst containing internal electron donor ID1) in the polymerization experiment was replaced with the solid catalyst component 2 (i.e., Z-N catalyst containing internal electron donor ID2).

### Olefin Polymerization Example b2

Propylene polymerization was carried out in accordance with Polymerization Example b1, except that the solid catalyst component 1 (i.e., Z-N catalyst containing internal electron donor ID1) in the polymerization experiment was replaced with the solid catalyst component 2 (i.e., Z-N catalyst containing internal electron donor ID2).

The resultant polypropylene product was characterized to determine its polymer properties, and the test results are shown in Table 2.

### Catalyst Preparation Example 3

The synthetic procedure for the solid catalyst component was identical to that described in Catalyst Preparation Example 1, except that the internal electron donor ID1 was replaced with ID3. All remaining operating steps were kept consistent with Catalyst Preparation Example 1 to give solid catalyst component 3 (i.e., Z-N catalyst containing internal electron donor ID3).

The contents of titanium and internal electron donor in solid catalyst component 3, and the structure of the internal electron donor are shown in Tables 1 and 2 below.

### Olefin Polymerization Example a3

Propylene polymerization was carried out in accordance with Polymerization Example a1, except that the solid catalyst component 1 (i.e., Z-N catalyst containing internal electron donor ID1) in the polymerization experiment was replaced with the solid catalyst component 3 (i.e., Z-N catalyst containing internal electron donor ID3).

### Olefin Polymerization Example b3

Propylene polymerization was carried out in accordance with Polymerization Example b1, except that the solid catalyst component 1 (i.e., Z-N catalyst containing internal electron donor ID1) in the polymerization experiment was replaced with the solid catalyst component 3 (i.e., Z-N catalyst containing internal electron donor ID3).

The resultant polypropylene product was characterized to determine its polymer properties, and the test results are shown in Table 2.

### Catalyst Preparation Example 4

The synthetic procedure for the solid catalyst component was identical to that described in Catalyst Preparation Example 1, except that the internal electron donor ID1 was replaced with ID4. All remaining operating steps were kept consistent with Catalyst Preparation Example 1 to give Solid catalyst component 4 (i.e., Z-N catalyst containing internal electron donor ID4).

The contents of titanium and internal electron donor in the solid catalyst component 4, and the structure of the internal electron donor are shown in Tables 1 and 2 below.

### Olefin Polymerization Example a4

Propylene polymerization was carried out in accordance with Polymerization Example a1, except that the solid catalyst component 1 (i.e., Z-N catalyst containing internal electron donor ID1) in the polymerization experiment was replaced with the solid catalyst component 4 (i.e., Z-N catalyst containing internal electron donor ID4).

### Olefin Polymerization Example b4

Propylene polymerization was carried out in accordance with Polymerization Example b1, except that the solid catalyst component 1 (i.e., Z-N catalyst containing internal electron donor ID1) in the polymerization experiment was replaced with the solid catalyst component 4 (i.e., Z-N catalyst containing internal electron donor ID4).

The resultant polypropylene product was characterized to determine its polymer properties, and the test results are shown in Table 2.

### Catalyst Preparation Example 5

The synthetic procedure for the solid catalyst component was identical to that described in Catalyst Preparation Example 1, except that the internal electron donor ID1 was replaced with ID5. All remaining operating steps were kept consistent with Catalyst Preparation Example 1 to give solid catalyst component 5 (i.e., Z-N catalyst containing internal electron donor ID5).

The contents of titanium and internal electron donor in solid catalyst component 5, and the structure of the internal electron donor are shown in Tables 1 and 2 below.

### Olefin Polymerization Example a5

Propylene polymerization was carried out in accordance with Polymerization Example a1, except that the solid catalyst component 1 (i.e., Z-N catalyst containing internal electron donor ID1) in the polymerization experiment was replaced with the solid catalyst component 5 (i.e., Z-N catalyst containing internal electron donor ID5).

### Olefin Polymerization Example b5

Propylene polymerization was carried out in accordance with Polymerization Example b1, except that the solid catalyst component 1 (i.e., Z-N catalyst containing internal electron donor ID1) in the polymerization experiment was replaced with the solid catalyst component 5 (i.e., Z-N catalyst containing internal electron donor ID5).

The resultant polypropylene product was characterized to determine its polymer properties, and the test results are shown in Table 2.

### Catalyst Preparation Example 6

The synthetic procedure for the solid catalyst component was identical to that described in Catalyst Preparation Example 1, except that the internal electron donor ID1 was replaced with ID6. All remaining operating steps were kept consistent with Catalyst Preparation Example 1 to give solid catalyst component 6 (i.e., Z-N catalyst containing internal electron donor ID6).

The contents of titanium and internal electron donor in the solid catalyst component 6, and the structure of the internal electron donor are shown in Tables 1 and 2 below.

### Olefin Polymerization Example a6

Propylene polymerization was carried out in accordance with Polymerization Example a1, except that the solid catalyst component 1 (i.e., Z-N catalyst containing internal electron donor ID1) in the polymerization experiment was replaced with the solid catalyst component 6 (i.e., Z-N catalyst containing internal electron donor ID6).

### Olefin Polymerization Example b6

Propylene polymerization was carried out in accordance with Polymerization Example b1, except that the solid catalyst component 1 (i.e., Z-N catalyst containing internal electron donor ID1) in the polymerization experiment was replaced with the solid catalyst component 6 (i.e., Z-N catalyst containing internal electron donor ID6).

The resultant polypropylene product was characterized to determine its polymer properties, and the test results are shown in Table 2.

### Catalyst Preparation Comparative Example 1

The synthetic procedure for the solid catalyst component was identical to that described in Catalyst Preparation Example 1, except that the internal electron donor ID1 was replaced with C1. All remaining operating steps were kept consistent with Catalyst Preparation Example 1 to give solid catalyst component 1' (i.e., Z-N catalyst containing internal electron donor C1).

The contents of titanium and internal electron donor in the solid catalyst component 1', and the structure of the internal electron donor are shown in Tables 1 and 2 below.

### Olefin Polymerization Comparative Example a1'

Propylene polymerization was carried out in accordance with Polymerization Example a1, except that the solid catalyst component 1 (i.e., Z-N catalyst containing internal electron donor ID1) in the polymerization experiment was replaced with the solid catalyst component 1' (i.e., Z-N catalyst containing internal electron donor C1).

### Olefin Polymerization Comparative Example b1'

Propylene polymerization was carried out in accordance with Polymerization Example b1, except that the solid catalyst component 1 (i.e., Z-N catalyst containing internal electron donor ID1) in the polymerization experiment was replaced with the solid catalyst component 1' (i.e., Z-N catalyst containing internal electron donor C1).

The resultant polypropylene product was characterized to determine its polymer properties, and the test results are shown in Table 2.

### Catalyst Preparation Comparative Example 2

The synthetic procedure for the solid catalyst component was identical to that described in Catalyst Preparation Example 1, except that the internal electron donor ID1 was replaced with C2. All remaining operating steps were kept consistent with Catalyst Preparation Example 1 to give solid catalyst component 2' (i.e., Z-N catalyst containing internal electron donor C2).

The contents of titanium and internal electron donor in the solid catalyst component 2', and the structure of the internal electron donor are shown in Tables 1 and 2 below.

### Olefin Polymerization Comparative Example a2'

Propylene polymerization was carried out in accordance with Polymerization Example a1, except that the solid catalyst component 1 (i.e., Z-N catalyst containing internal electron donor ID1) in the polymerization experiment was replaced with the solid catalyst component 2' (i.e., Z-N catalyst containing internal electron donor C2).

### Olefin Polymerization Comparative Example b2'

Propylene polymerization was carried out in accordance with Polymerization Example b1, except that the solid catalyst component 1 (i.e., Z-N catalyst containing internal electron donor ID1) in the polymerization experiment was replaced with the solid catalyst component 2' (i.e., Z-N catalyst containing internal electron donor C2).

The resultant polypropylene product was characterized to determine its polymer properties, and the test results are shown in Table 2.

### Catalyst Preparation Comparative Example 3

The synthetic procedure for the solid catalyst component was identical to that described in Catalyst Preparation Example 1, except that the internal electron donor ID1 was replaced with C3. All remaining operating steps were kept consistent with Catalyst Preparation Example 1 to give solid catalyst component 3' (i.e., Z-N catalyst containing internal electron donor C3).

The contents of titanium and internal electron donor in solid catalyst component 3', and the structure of the internal electron donor are shown in Tables 1and 2 below.

### Olefin Polymerization Comparative Example a3'

Propylene polymerization was carried out in accordance with Polymerization Example a1, except that the solid catalyst component 1 (i.e., Z-N catalyst containing internal electron donor ID1) in the polymerization experiment was replaced with the solid catalyst component 3' (i.e., Z-N catalyst containing internal electron donor C3).

### Olefin Polymerization Comparative Example b3'

Propylene polymerization was carried out in accordance with Polymerization Example b1, except that the solid catalyst component 1 (i.e., Z-N catalyst containing internal electron donor ID1) in the polymerization experiment was replaced with the solid catalyst component 3' (i.e., Z-N catalyst containing internal electron donor C3).

The resultant polypropylene product was characterized to determine its polymer properties, and the test results are shown in Table 2.

The polymer products from the Polymerization Examples and Comparative Examples were characterized in accordance with the test procedures specified below:
1. Melt flow rate (MFR): measured according to ASTM D1238;
2. Isotactic index: determined with an automatic xylene-soluble fraction analyzer (CRYSTEX QC) from Polymer Char Company. The value of the isotactic index was calculated by subtracting the measured content of the xylene-soluble fraction from 1;
3. Molecular weight and molecular weight distribution: determined using the High-temperature gel permeation chromatograph (GPC-IR6) from Polymer Char Company;
4. Catalytic activity: As commonly known in this field, catalyst activity is calculated by dividing the mass of collected polymer by the initial catalyst amount or via other conventional calculation routes.

**Table 1**

| Cataly st Examp les | Internal electron donor ID | Content of ID (wt%) | Content of Ti (wt%) | Polym erizati on Examp les | Amount of Hydrogen (g) | Activity (Kg/g Cat.h⁻¹) |
|---|---|---|---|---|---|---|
| Examp le 1 | 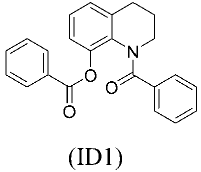 | 9.7 | 3.5 | Examp le a1 | 0.2 | 42.5 |
| | | | | Examp le b1 | 1.2 | 41.9 |
| Examp le 2 | 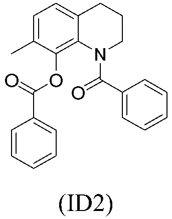 | 12.4 | 3.2 | Examp le a2 | 0.2 | 45.1 |
| | | | | Examp le b2 | 1.2 | 42.6 |
| Examp le 3 | 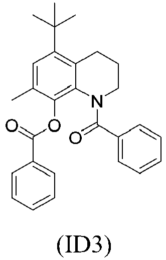 | 10.2 | 3.7 | Examp le a3 | 0.2 | 44.8 |
| | | | | Examp le b3 | 1.2 | 40.4 |
| Examp le 4 | 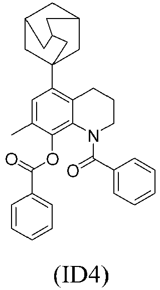 | 14.7 | 3.1 | Examp le a4 | 0.2 | 45.2 |
| | | | | Examp le b4 | 1.2 | 40.7 |
| Examp le 5 | 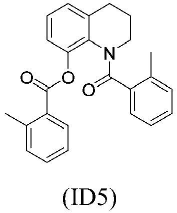 | 10.1 | 3.4 | Examp le a5 | 0.2 | 43.6 |
| | | | | Examp le b5 | 1.2 | 43.2 |
| Examp le 6 | 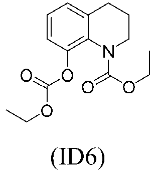 | 11.7 | 3.1 | Examp le a6 | 0.2 | 39.2 |
| | | | | Examp le b6 | 1.2 | 38.5 |
| Compa rative Examp le 1 | DNBP (C1) | 13.5 | 2.7 | Compa rative Examp le a1' | 0.2g | 37.1 |
| | | | | Compa rative Examp le b1' | 1.2g | 35.6 |
| Compa rative Examp le 2 | 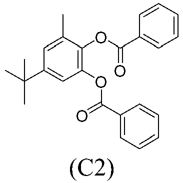 | 13.2 | 3.5 | Compa rative Examp le a2' | 0.2g | 42.2 |
| | | | | Compa rative Examp le b2' | 1.2g | 40.1 |
| Compa rative Examp le 3 | 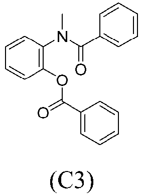 | 9.3 | 2.9 | Compa rative Examp le a3' | 0.2g | 39.5 |
| | | | | Compa rative Examp le b3' | 1.2g | 40.1 |

**Table 2**

| Catalyst Examples | Internal electron donor ID | Polymerization Examples | Isotactic index (I.I.) (wt%) | Melt Flow Rate (MFR) (g/10 min) | Molecular weight distribution |
|---|---|---|---|---|---|
| Example 1 | 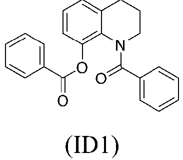 | Example a1 | 98.3 | 3.8 | 6.7 |
| | | Example b1 | 97.9 | 97.3 | 6.9 |
| Example 2 | 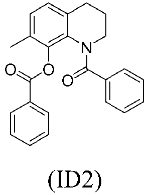 | Example a2 | 98.5 | 4.1 | 6.5 |
| | | Example b2 | 98.0 | 110.8 | 6.3 |
| Example 3 | 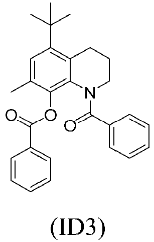 | Example a3 | 98.8 | 3.6 | 7.0 |
| | | Example b3 | 98.1 | 127.6 | 6.6 |
| Example 4 | 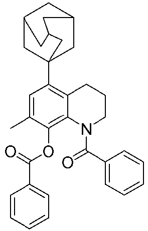 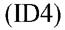 | Example a4 | 98.9 | 3.7 | 6.8 |
| | | Example b4 | 98.3 | 145.3 | 6.5 |
| Example 5 | 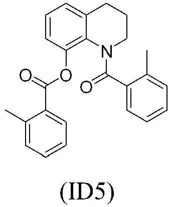 | Example a5 | 98.2 | 3.9 | 7.1 |
| | | Example b5 | 97.7 | 100.3 | 6.8 |
| Example 6 | 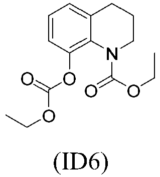 | Example a6 | 98.5 | 4.2 | 6.8 |
| | | Example b6 | 97.8 | 89.5 | 6.3 |
| Comparative Example 1 | 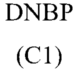 | Comparative Example a1' | 98.5 | 3.2 | 5.6 |
| | | Comparative Example b1' | 97.8 | 43.96 | 5.2 |
| Comparative Example 2 | 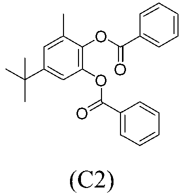 | Comparative Example a2' | 98.5 | 4.0 | 6.4 |
| | | Comparative Example b2' | 98.0 | 102.9 | 6.6 |
| Comparative Example 3 | 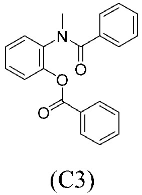 | Comparative Example a3' | 98.1 | 3.7 | 6.3 |
| | | Comparative Example b3' | 97.2 | 67.2 | 6.1 |

The results in the above table show that when the catalysts using hydrogenated 8-hydroxyquinoline derivatives as internal electron donors are used for propylene polymerization, the catalysts exhibit the characteristics of high polymerization activity and high hydrogen response, and the prepared polymers have high isotactic indexes and broad molecular weight distribution. Under the same preparation process conditions, compared with the catalysts using other noncyclic aromatic amine compounds as internal electron donors, the catalysts using hydrogenated 8-hydroxyquinoline derivatives as internal electron donors exhibit higher hydrogen response, and the prepared polymers have higher isotactic indexes. In addition, the molecular weight distribution of the polypropylene obtained by the present disclosure is generally broader than that obtained by using phthalate ester catalysts.

Examples of the present disclosure have been described above, the above explanations are exemplary, but not exhaustive, and are not limited to the disclosed Examples. Without deviating from the scope and spirit of the explained Examples, various modifications and changes are obvious to those of ordinary skill in the art.

## Claims

1. An internal electron donor for an olefin polymerization catalyst, wherein the internal electron donor is at least one selected from the compounds represented by Formula I below: 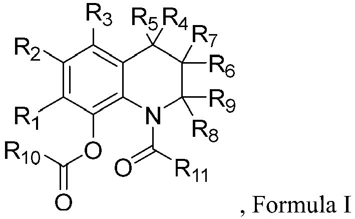 in Formula I:
R₁ to R₉ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl; two or more of R₁ to R₉ may be linked together to form cyclic structure(s) or unsaturated bond(s);
R₁₀ and R₁₁ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl;
any hydrogen atom on benzene ring of aryl, arylalkyl, or alkylaryl group may each be optionally substituted with a halogen atom.

2. The internal electron donor according to claim 1, wherein the internal electron donor is at least one selected from the compounds represented by Formula (II) below: 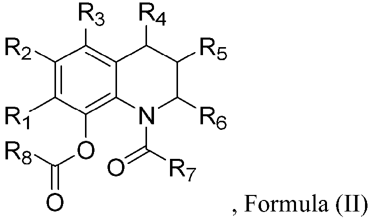 in Formula (II):
R₁ to R₆ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl; two or more of R₁ to R₆ may be linked together to form cyclic structure(s) or unsaturated bond(s);
R₇ and R₈ are the same or different, and each is independently selected from H, halogen, saturated or unsaturated linear or branched C₁-C₁₀ alkyl, cycloalkyl, alkenyl, ester group, phenyl, alkylphenyl, phenylalkyl, indenyl and benzyl; halo-substituted or heteroatom-substituted alkyl, cycloalkyl, phenyl, alkylphenyl, phenylalkyl, indenyl and benzyl, wherein the heteroatom is selected from N, O, S, P and Si; and heteroaryl.

3. The internal electron donor according to claim 2, wherein in Formula (II), each of R₇ and R₈ is independently selected from phenyl, tert-butyl and ethoxy.

4. A solid catalyst component for olefin polymerization comprising: a titanium compound, a magnesium compound, and at least one of the internal electron donors of any one of claims 1 to 3.

5. The solid catalyst component according to claim 4, wherein a method for preparing the solid catalyst component comprises: bring a magnesium compound precursor, the titanium compound and at least one of the internal electron donors into contact and reaction to obtain the solid catalyst component; wherein,
the magnesium compound precursor is at least one selected from the compounds represented by Formula XₙMg(OR)₂₋ₙ, the compounds represented by Formula MgCl₂·mROH, MgCl₂/SiO₂ mixtures, MgCl₂/Al₂O₃ mixtures, and mixtures of magnesium halides and titanium alkoxides; wherein m ranges from 0.1 to 6, n is no less than 0 and no more than 2, X is selected from halogen, R is selected from hydrogen and C₁-C₈ hydrocarbyl group;
the titanium compound is represented by Formula TiXₙ(OR)₄₋ₙ, wherein R is selected from C₁-C₂₀ hydrocarbyl, X is selected from halogen, and n ranges from 1 to 4.

6. A polymerization catalyst for olefin polymerization, wherein the polymerization catalyst comprises a product obtained by reacting the following starting materials:
(a) at least one of the solid catalyst components of claim 4 or 5;
(b) at least one of the organo-aluminum compounds represented by Formula AlRₙX₍₃₋ₙ₎, wherein R is selected from hydrogen and C₁-C₂₀ hydrocarbyl group; X is selected from halogen, and n is an integer satisfying 0≦n≦3;
(c) at least one of alkoxysilane compounds represented by Formula RₙSi(OR¹)₄₋ₙ, wherein R and R¹ are the same or different, and each is independently selected from C₁-C₁₈ hydrocarbyl group and halogenated hydrocarbyl group, and C₁-C₁₀ substituent optionally containing heteroatom(s); and n is an integer satisfying 0≦n≦3.

7. The polymerization catalyst according to claim 6, wherein, for the organo-aluminum compounds represented by Formula AlRₙX₍₃₋ₙ₎, each R is hydrogen or a C₁-C₁₂ hydrocarbyl group.

8. The polymerization catalyst according to claim 6 or 7, wherein, the organo-aluminum compound is a trialkylaluminum compound, preferably one or more selected from trimethylaluminum, triethylaluminum, triisobutylaluminum, tri-n-butylaluminum, tri-n-hexylaluminum, and trioctylaluminum.

9. The polymerization catalyst according to any one of claims 6 to 8, wherein for the alkoxysilane compound represented by Formula RₙSi(OR¹)₄₋ₙ, R and R¹ are each independently selected from C₁-C₁₈ alkyl, C₃-C₁₈ cycloalkyl, C₆-C₁₈ aryl, and C₁-C₁₈ haloalkyl.

10. Use of the polymerization catalyst of any one of claims 6 to 9 in olefin polymerization.
